# EUROPÄISCHE PATENTANMELDUNG

(11) **EP 4 454 616 A1**
(43) Veröffentlichungstag der Anmeldung: **30.10.2024**
(21) Anmeldenummer: 23169657.6
(22) Anmeldetag: 25.04.2023
(51) Int. Cl.: A61F 2/38, A61F 2/30

(54) **IMPLANTIERBARE PROTHESE ZUM ERSATZ DES MENSCHLICHEN KNIEGELENKS**

(71) Anmelder: Baumgart, Rainer, 80539 München (DE)
(72) Erfinder: Baumgart, Rainer, 80539 München (DE)
(74) Vertreter: Lambacher, Michael

(57) **Zusammenfassung**

Es wird eine implantierbare Prothese (1) zum Ersatz eines menschlichen Kniegelenks bereitgestellt, die eine Gelenkeinrichtung (100) mit einem Gelenkkopf (20) und einem mit dem Gelenkkopf (20) zusammenwirkenden Gelenkfuß (40) umfasst, die zur Realisierung einer Gelenkbewegung in Form einer Beuge- und/oder Streckbewegung und wahlweise zusätzlich zur Realisierung einer dazu senkrechten eingeschränkten Drehbewegung vorgesehen sind. Der Gelenkkopf (20) weist zwei erste Gleitflächen (25) auf, die an gegenüberliegenden axialen Enden (24) des Gelenkkopfs (20) angeordnet sind und durch einen mittig im Gelenkkopf (20) angeordneten Innenraum (21) räumlich voneinander getrennt sind. Der Gelenkfuß (40) weist zwei zweite Gleitflächen (45) auf, die an dem Gelenkfuß (40) axial gegenüberliegend ausgebildet sind und mit jeweiligen ersten Gleitflächen (25) des Gelenkkopfs (20) in Berührung kommen, wobei die zwei ersten Gleitflächen (25) und die zwei zweiten Gleitflächen (45) zur Realisierung wenigstens einer Beuge- und/oder Streckbewegung ausgebildet sind.

## Beschreibung

### GEBIET DER ERFINDUNG

Die Erfindung betrifft eine implantierbare Prothese zum Ersatz des menschlichen Kniegelenks, sowie gegebenenfalls auch der angrenzenden Knochenabschnitte.

### HINTERGRUND

Bösartige Knochentumore sind häufig kniegelenksnah am Femur oder an der Tibia lokalisiert, so dass nach Entfernung des betroffenen Knochenabschnittes sowohl ein Teil des Schaftes als auch der dazu gehörige femorale oder tibiale Gelenkanteil durch eine implantierbare Prothese ersetzt werden müssen.

Der entfernte Teil des Schaftes des betroffenen Knochens wird durch das Knochenschaftersatzelement der Prothese ersetzt. Der Gelenkanteil des betroffenen Knochens wird durch den Gelenkanteil der Prothese ersetzt. In jedem Fall muss auch in den korrespondierenden Knochen ein Gelenkanteil implantiert werden, um eine Artikulation und eine Kopplung mit dem Gelenkanteil des betroffenen Knochens zu erreichen. Die Kopplung der Gelenkanteile erfolgt durch eine Gelenkeinrichtung, wie z.B. einen Scharnier- oder Kugelmechanismus, der eine Beweglichkeit zwischen Femur und Tibia ermöglichen soll, die der natürlichen Beweglichkeit des Kniegelenks möglichst nahekommt. Die Verankerung der Prothese im Femur und der Tibia erfolgt jeweils durch eine in den Knochen eingebrachte Schaftverankerung. Falls eine spätere Entfernung vorgesehen ist, kann die Schaftverankerung mit glatter Oberfläche ausgeführt sein, so dass kein Knochen einwächst. Falls eine definitive Verankerung vorgesehen ist, kann die Oberfläche aufgeraut oder beschichtet sein, so dass Knochengewebe einwachsen kann. Alternativ kann die Schaftverankerung auch mit Knochenzement fixiert werden.

Da bösartige Knochentumore häufig im Wachstumsalter auftreten, kommt es im weiteren Verlauf nach der chirurgischen Entfernung des Tumors und der Implantation einer Tumorprothese durch den Wegfall der Wachstumsfuge auf der betroffenen Seite und durch das weitere Wachstum der Gegenseite zu einer Beinlängendifferenz. Das verzögerte Wachstum betrifft zwar in erster Linie den von dem Tumor selbst befallenen aber auch den korrespondierenden Knochen, d.h. wenn der Tumor z.B. am Femur kniegelenksnah lokalisiert war, bleibt nicht nur das Femur sondern auch die Tibia im Wachstum zurück. Selbst wenn in diesem Fall die Schaftverankerung der Tibia glatt und poliert ausgeführt ist, so dass der Knochen grundsätzlich weiter wachsen kann, kommt es somit auch in dem korrespondierenden Knochen zu einer Wachstumsverzögerung, die zu der entstehenden Beinlängendifferenz beiträgt.

Möglichkeiten, den nach Tumorentfernung verbliebenen Knochen trotz Tumorprothese zu verlängern, sind aus der EP 1 371 346 A1 und der EP 2 468 216 A1 bekannt. In der EP 1 371 346 A1 wird hierzu durch die Prothese in den verbliebenen Knochen ein Austausch der Schaftverankerung gegen einen Verlängerungsmarknagel offenbart, der nach einer Osteotomie die beiden Knochenfragmente langsam auseinanderzieht, so dass in dem sich vergrößernden Spalt neues Knochengewebe entstehen kann (Kallusdistraktionsmethode). Bei Lokalisation im proximalen Femur erfolgt der Austausch ebenfalls von proximal, bei Lokalisation im distalen Femur erfolgt der Austausch ebenfalls von distal, wobei anatomisch bedingt nicht zwangsläufig eine Entkopplung der Prothese notwendig ist, da die Eintrittsstelle bei Kniebeugung axial für Werkzeuge und die Schaftverankerung oder auch einen Distraktionsmarknagel erreichbar ist. Bei Lokalisation des Tumors in der proximalen Tibia oder bei Verlängerungsbedarf der Tibia ist bedingt durch die Überdeckung des axialen Zugangs durch den femoralen Gelenkanteil der Prothese sowohl bei Streckung als auch bei Beugung ein Austausch der tibialen Verankerung ohne Entkopplung der Prothese nicht möglich.

Die EP 2 468 216 A1 offenbart erstmals eine Lösung, am Femur einen Austausch der Schaftverankerung in minimal invasiver Operationstechnik durchzuführen, indem der Wechsel der Komponenten von dem gegenüberliegenden Knochenende aus erfolgt. Sofern der Tumor am Femur lokalisiert ist und keine Längendifferenz am Unterschenkel besteht, kann somit mit einer Prothese der in der EP 2 468 216 A1 beschriebenen Art ein erneuter großer Zugang über die Prothese vermieden und damit das Infektionsrisiko deutlich gesenkt werden.

Bei Verlängerungsbedarf der Tibia, sei es nach primärem Tumorbefall der Tibia oder wenn nach Tumorlokalisation am Femur auch die Tibia verkürzt ist und deshalb die vorläufige Schaftverankerung in der Tibia gegen einen Verlängerungsmarknagel und nach Wachstumsende der Verlängerungsmarknagel gegen eine beschichtete Schaftverankerung ausgetauscht werden soll, die in den Knochen dauerhaft einwächst, muss jedoch weiterhin jedes Mal die Prothese entkoppelt werden, wozu ein großer operativer Zugang mit einem erheblichen Infektionsrisiko erforderlich ist.

Der in der EP 2 468 216 A1 beschriebene Vorteil kommt somit letztendlich nur dann zum Tragen, wenn nicht gleichzeitig auch eine Unterschenkelverkürzung vorliegt, da die in der EP 2 468 216 A1 beschriebene Lösung am Unterschenkel nicht anwendbar ist. Für den häufigen Fall, dass die Verkürzung aber, so wie bereits erwähnt, auch den Unterschenkel betrifft, besteht nach der in EP 1 371 346 A1 beschriebenen Technik keine Möglichkeit, eine Verlängerung ohne Entkopplung der Prothese in minimal invasiver Technik durchzuführen. Somit steht den vorteilhaften Möglichkeiten eines Beinlängenangleichs an die Gegenseite durch Knochenwachstum weiterhin das erhebliche Infektionsrisiko durch wiederholte große operative Zugänge im Bereich der Prothese gegenüber.

Dieses Problem wird durch die in der EP 3 135 253 A1 offenbarte Prothese adressiert. Die dort beschriebene Prothese weist einen femoralen Gelenkanteil sowie einen tibialen Gelenkanteil auf, die jeweils Durchgangsöffnungen aufweisen und die über eine Gelenkeinrichtung gelenkig miteinander verbunden sind. Die Durchgangsöffnungen entsprechen einem Führungskanal, der je nach Kniebeugung einmal mit dem femoralen Gelenkanteil und einmal mit dem tibialen Gelenkanteil fluchtet und somit die Durchführung von Werkzeugen, einer stabförmigen Schaftverankerung oder eines Distraktionsmarknagels von außen sowohl in das Femur als auch in die Tibia ermöglicht, ohne dass die Prothese entkoppelt werden muss.

Der hier vorliegenden Erfindung liegt die Aufgabe zugrunde, eine Prothese zum Ersatz des menschlichen Kniegelenks bereitzustellen, welche einerseits die Vorteile der in der EP 3 135 253 A1 beschriebenen Prothese aufweist und andererseits eine den hohen biomechanischen Anforderungen entsprechende, verbesserte, insbesondere stabilere Gelenkeinrichtung aufweist.

### KURZER ABRISS

Zur Lösung der oben genannten Aufgabe sowie weiterer Aufgaben wird eine implantierbare Prothese zum Ersatz eines menschlichen Kniegelenks bereitgestellt. Die Prothese umfasst eine Gelenkeinrichtung mit einem Gelenkkopf und einen mit dem Gelenkkopf zusammenwirkenden Gelenkfuß, die im Wesentlichen zur wahlweisen Realisierung einer Gelenkbewegung in Form einer Beuge- und/oder Streckbewegung oder auch einer eingeschränkten Rotationsbewegung um die Tibiaschaftachse vorgesehen sind. Der Gelenkkopf der Prothese weist zwei erste Gleitflächen auf, die an gegenüberliegenden axialen Enden des Gelenkkopfs angeordnet sind und durch einen mittig im Gelenkkopf angeordneten Innenraum räumlich voneinander getrennt sind. Der Gelenkfuß weist zwei zweite Gleitflächen auf, die an dem Gelenkfuß axial gegenüberliegend ausgebildet sind und mit den jeweiligen ersten Gleitflächen des Gelenkkopfs in Berührung kommen, wobei die zwei ersten Gleitflächen und die zwei zweiten Gleitflächen zur Realisierung wenigstens einer Beuge- und/oder Streckbewegung ausgebildet sind.

Der Gelenkkopf sowie der mit dem Gelenkkopf zusammenwirkende Gelenkfuß definieren eine Gelenkachse der Gelenkeinrichtung, um welche der Gelenkfuß bezüglich des Gelenckopfs bewegbar (drehbar) ist, um eine Beuge- bzw. Streckbewegung ausführen zu können. Der Gelenkkopf und der Gelenkfuß sind dabei derart ausgestaltet, dass die Gelenkachse der Gelenkeinrichtung in Relation zur Mitte der Femurlängsachse (geringfügig) in dorsaler Richtung verlagert ist. Die Gelenkachse definiert die axiale Richtung der Gelenkeinrichtung. Mit anderen Worten ist mit axialer Richtung die Richtung entlang der Gelenkachse gemeint. Entsprechend ist mit "axialem Ende" oder "axialen Enden" des Gelenkkopfs jenes Ende bzw. jene Enden (Endflächen) gemeint, welche den Gelenkkopf entlang seiner axialen Achse begrenzen.

Gemäß einer Implementierung kann der Gelenkkopf der Prothese im Wesentlichen walzenförmig (zylinderförmig) oder hohlzylinderförmig ausgebildet sein. Der walzenförmige oder hohlzylinderförmige Gelenkkopf kann eine vorgegebene axiale Ausdehnung (also Ausdehnung entlang seiner Rotationsachse) und eine vorgegebene radiale Ausdehnung (Ausdehnung senkrecht zu seiner Rotationsachse) aufweisen. Entsprechend können die zwei ersten Gleitflächen an oder in der Nähe der gegenüberliegenden axialen Enden des walzenförmigen oder hohlzylinderförmigen Gelenkkopfs ausgebildet sein.

Der Innenraum kann in axialer Richtung mittig im Gelenkkopf ausgebildet sein. Das bedeutet, dass der Innenraum von den jeweiligen gegenüberliegenden axialen Enden des walzenförmigen oder holzylinderförmigen Gelenkkopfs (im Wesentlichen) denselben axialen Abstand aufweist. Insbesondere kann der Innenraum als Aussparung mit einer in radialer Richtung ausgebildeten Austrittsöffnung ausgebildet sein.

Die Prothese kann ferner einen Formkörper umfassen, der derart ausgestaltet ist, dass er im Innenraum bzw. in der Aussparung (passgenau) formschlüssig aufgenommen werden kann. Insbesondere kann der Formkörper derart ausgestaltet sein, dass er auch nach der Kopplung der Prothese entnehmbar in den Innenraum bzw. in die Aussparung einsetzbar ist, da er keine biomechanische Funktion bei der Kopplung der Gelenkanteile übernimmt, sondern lediglich den Innenraum ausfüllt, einen Teil der patellaren Gleitfläche übernimmt und wenigstens einen Führungskanal, bevorzugt zwei Führungskanäle aufweisen kann.

Gemäß einer Variante kann (können) der wenigstens eine Führungskanal (die zwei Führungskanäle jeweils) als Durchgangsbohrung im Formkörper ausgebildet sein. Der wenigstens eine Führungskanal (Durchgangsbohrung) kann hierbei derart dimensioniert sein, dass ein Werkzeug, ein Distraktionsmarknagel und/oder eine femorale oder tibiale Schaftverankerung durch den wenigstens einen Führungskanal hindurchführbar ist. Insbesondere kann der wenigstens eine Führungskanal derart im Formkörper angeordnet (bzw. ausgerichtet) sein, dass der wenigstens eine Führungskanal mit einer Durchgangsöffnung eines angrenzenden femoralen oder tibialen Gelenkanteils in Flucht kommt, wenn die Gelenkeinrichtung einen vorgegebenen Beugungswinkel realisiert. Bei dem angrenzenden femoralen Gelenkanteil kann es sich um den Gelenkkopf und bei dem angrenzenden tibialen Gelenkanteil um den Gelenkfuß der Gelenkeinrichtung handeln. Weist der Formkörper zwei Führungskanäle (Durchgangsbohrungen) auf, so können die beiden Kanäle im Formkörper kreuzend zueinander derart angeordnet sein, dass der erste Führungskanal mit einer Durchgangsöffnung eines angrenzenden femoralen Gelenkanteils und der zweite Führungskanal mit einer Durchgangsöffnung eines angrenzenden tibialen Gelenkanteils in Flucht kommt, wenn die Gelenkeinrichtung einen vorgegebenen Beugungswinkel realisiert. Der vorgegebene Beugungswinkel kann hierbei einem Winkel entsprechen, welchen der Gelenkkopf und der Gelenkfuß der Gelenkeinrichtung bei der Realisierung einer einer Kniebeugung entsprechenden Funktionsstellung einnehmen.

Ähnlich wie in der EP 3 135 253 A1 kann der vorgegebene Beugungswinkel, bei dem der wenigstens eine Führungskanal (bzw. zwei Führungskanäle) des Formkörpers mit einer Durchgangsöffnung eines angrenzenden femoralen und/oder tibialen Gelenkanteils (Gelenkkopfs und/oder Gelenkfußes) fluchtet, im Bereich von 20° bis 90°, vorzugsweise 30° bis 60°, besonders bevorzugt 30° bis 45° liegen. Durch die hier beschriebene Ausbildung wenigstens eines Führungskanals im Formkörper, kann auf einfache und schonende Weise ein Werkzeug, ein Distraktionsmarknagel oder eine femorale oder tibiale Schaftverankerung über den wenigstens einen Führungskanal in die angrenzende Durchgangsöffnung des femoralen und/oder tibialen Gelenkersatzes eingeführt werden, ohne dass die Gelenkeinrichtung der Prothese entkoppelt werden muss. Dadurch kann der operative Eingriff (beispielsweise beim Ersatz eines Distraktionsmarknagels durch eine tibiale Schaftverankerung) erheblich reduziert und das Infektionsrisiko deutlich gesenkt werden.

Der Gelenkfuß kann zur beweglichen Aufnahme des (walzenförmigen oder hohlzylinderförmigen) Gelenkkopfs ausgebildet sein. Insbesondere kann der Gelenkfuß gabelförmig ausgebildet sein mit zwei seitlich (medial und lateral) angeordneten Gelenkelementen, die axial voneinander beabstandet angeordnet sind. Die seitlichen Gelenkelemente können im Vergleich zu den axialen Enden des Gelenkkopfs axial weiter außen angeordnet sein. Ferner können die zwei zweiten Gleitflächen des Gelenkfußes an den Innenseiten der zwei seitlichen Gelenkelemente angeordnet sein, so dass jeweils eine zweite Gleitfläche mit einer entsprechenden ersten Gleitfläche am axialen Ende des Gelenkkopfs gleitend zusammenwirken kann. Mit anderen Worten sind der Gelenkkopf und der Gelenkfuß über ihre jeweiligen axial außenliegenden Gleitflächen um die Gelenkachse drehbar gelagert. Diese gabelförmige Außenlagerung von Gelenkkopf und Gelenkfuß erhöht die Stabilität der Gelenkeinrichtung. Gleichzeitig weist die Gelenkeinrichtung im Zentrum keine zur gelenkigen Verbindung beitragenden Gelenkkomponenten auf. Der im Zentrum vorgesehene Innenraum (Aussparung) steht somit nicht im Kraftfluss. Entsprechend ist auch der in der Aussparung einsetzbare Formkörper nicht im Kraftfluss. Ihm kommt somit keine Gelenkfunktion zu, sondern vor allem eine Führungsfunktion zur Einführung eines Werkzeugs, eines Distraktionsmarknagels und/oder einer femoralen oder tibialen Schaftverankerung. Im Gegensatz zu der in der EP 3 135 253 B1 beschriebenen Gelenkeinrichtung, sind mit der erfindungsgemäßen Gelenkeinrichtung die Gelenkfunktion und der damit einhergehende Kraftfluss von der Führungsfunktion für die Verankerungselemente räumlich und funktionell vollständig entkoppelt.

Die zwei ersten Gleitflächen können jeweils radial gekrümmte Gleitflächen sein. Insbesondere können die zwei ersten Gleitflächen in Umfangsrichtung jeweils einen vorgegebenen ersten radialen Abstand von der Gelenkachse (Rotationsachse des Gelenkkopfs) aufweisen. Sie können somit die Krümmung eines Kreises mit einem vorgegebenen ersten Radius aufweisen. Ebenso können die zwei zweiten Gleitflächen jeweils radial gekrümmte Gleitflächen sein. Insbesondere können die zwei zweiten Gleitflächen in Umfangsrichtung einen vorgegebenen zweiten radialen Abstand von der Gelenkachse (Rotationsachse des Gelenkkopfs) aufweisen. Sie können somit die Krümmung eines Kreises mit einem vorgegebenen zweiten Radius aufweisen.

Beispielsweise können die jeweiligen ersten Gleitflächen die Innenfläche eines jeweiligen Außenringes sein, der an den jeweiligen gegenüberliegenden axialen Enden des Gelenkkopfs axial nach außen vorsteht. Ferner können die jeweiligen zweiten Gleitflächen die Außenfläche eines jeweiligen Innenringes sein, der an den jeweiligen gegenüberliegenden seitlichen Gelenkelementen axial nach innen vorsteht. Alternativ können die jeweiligen ersten Gleitflächen die Außenfläche eines jeweiligen Innenringes sein, der an den jeweiligen gegenüberliegenden axialen Enden des Gelenkkopfs axial nach außen vorsteht. Ferner können die jeweiligen zweiten Gleitflächen Innenflächen eines jeweiligen Außenringes sein, der an den jeweiligen gegenüberliegenden seitlichen Gelenkelementen axial nach innen vorsteht. Im montierten Zustand der Gelenkeinrichtung greifen an beiden axialen Enden die jeweiligen Innenringe in die jeweiligen Außenringe ein, wodurch die ersten Gleitflächen jeweils mit den zweiten Gleitflächen gleitend in Berührung kommen. Durch die hier beschriebene kreisförmig gekrümmte Ausbildung der ersten Gleitflächen und zweiten Gleitflächen relativ zur Gelenkachse kann eine Drehbewegung um die Gelenkachse der Gelenkeinrichtung und somit eine wahlweise Beuge- und/oder Streckbewegung realisiert werden.

Alternativ können die ersten Gleitflächen und die zweiten Gleitflächen auch sphärisch oder zumindest teilweise sphärisch ausgebildet sein. Gemäß einer Variante können die zwei ersten Gleitflächen jeweils (teilweise) sphärisch konvex und die korrespondierenden zweiten Gleitflächen jeweils (teilweise) sphärisch konkav ausgebildet sein und somit jeweils ein Teilsegment einer Kugeloberfläche bilden. Beispielsweise können die (teilweise) sphärisch konvex ausgebildeten ersten Gleitflächen an den gegenüberliegenden axialen Enden des Gelenkkopfs vorstehen, während die (teilweise) sphärisch konkav ausgebildeten zweiten Gleitflächen an den Innenseiten der axial gegenüberliegenden seitlichen Gelenkelelemente des Gelenkfußes eingelassen sind. Gemäß einer alternativen Variante können die zwei ersten Gleitflächen jeweils (teilweise) sphärisch konkav und die korrespondierenden zweiten Gleitflächen jeweils (teilweise) sphärisch konvex ausgebildet sein. In diesem Fall können die (teilweise) sphärisch konkav ausgebildeten ersten Gleitflächen an den gegenüberliegenden axialen Enden des Gelenkkopfs eingelassen sein, während die (teilweise) sphärisch konvex ausgebildeten zweiten Gleitflächen an den Innenseiten der axial gegenüberliegenden seitlichen Gelenkelemente des Gelenkfußes hervorstehen.

Die (teilweise) sphärisch konvex ausgebildeten Gleitflächen können die Oberflächen entsprechender Vorsprünge sein, die entweder an den Innenseiten der seitlichen Gelenkelemente des Gelenkfußes oder an den axialen Enden (axialen Außenseiten) des Gelenkkopfs ausgebildet sind. Ferner können die (teilweise) sphärisch konkav ausgebildeten Flächen die Oberflächen entsprechender Nuten sein, die entweder an den Innenseiten der seitlichen Gelenkelelemente des Gelenkfußes oder an den axialen Enden (axialen Außenseiten) des Gelenkkopfs ausgebildet sind. Im montierten Zustand der Gelenkeinrichtung greifen an beiden axialen Enden die jeweiligen (teilweise) konvex ausgebildeten sphärischen Gleitflächen in die entsprechenden, jeweils (teilweise) konkav ausgebildeten sphärischen Gleitflächen ein, wodurch zwischen dem Gelenkkopf und dem Gelenkfuß eine (partielle) Kugellagerung realisiert wird, welche nicht nur eine Drehbewegung um die Gelenkachse zur Realisierung einer Beuge- und Streckbewegung sondern zusätzlich noch eine begrenzte Drehbewegung (Rotationsbewegung um einige Grad) senkrecht zur Gelenkachse ermöglicht. Dadurch kann die natürliche Beweglichkeit eines Kniegelenkes noch besser nachgebildet werden.

Unabhängig von der hier beschriebenen konkreten Ausgestaltung können die zwei ersten Gleitflächen und/oder die zwei zweiten Gleitflächen jeweils eine biokompatible Beschichtung zur Reibungsverminderung aufweisen. Alternativ können die zwei ersten Gleitflächen und/oder zwei zweiten Gleitflächen jeweils aus einer zusätzlichen Materialkomponente gefertigt sein, welche verschleißresistent ist und zur Reibungsverminderung zwischen dem Gelenkkopf und dem Gelenkfuß beiträgt. Denkbar ist der Einsatz jeglicher Art von Materialpaarung bzw. Oberflächenbeschichtung für die ersten und zweiten Gleitflächen, wie sie aus dem Stand der Technik bekannt sind.

Ferner kann der femorale Gelenkanteil (Gelenkkopf) ventralseitig, gemeinsam mit dem innenliegenden Formkörper der Gelenkeinrichtung, eine Gleitfläche für eine Kniescheibe aufweisen.

Zur Begrenzung der Drehbewegung um die Gelenkachse, insbesondere der Streckbewegung, kann die Gelenkeinrichtung einen Streckanschlag aufweisen. Der Streckanschlag kann durch wenigstens eine nach ventral hin (also zur Vorderseite hin) ausgebildete Anschlagfläche am Gelenkkopf realisiert sein. Die wenigstens eine Anschlagfläche am Gelenkkopf kann dadurch realisiert werden, dass der walzenförmige oder hohlzylinderförmige Gelenkkopf in radialer Richtung eine Körperausdehnung aufweist, die zur Ventralseite des Gelenkkopfs hin lokal (leicht) zunimmt. Mit anderen Worten ist der Gelenkkopf an seiner Außenseite in Umfangsrichtung nicht vollständig kreisrund und konzentrisch zu dem kreisförmigen oder kreisförmig/sphärischen Innenradius ausgebildet, sondern weist eine vom kreisrunden Verlauf abweichende, und zur Ventralseite hin gerichtete lokale Materialzunahme auf. Durch diese ventralseitige, lokale Materialzunahme (Ausdehnungszunahme) des Gelenkkopfs wird eine Anschlagfläche geschaffen, die mit einem entsprechenden Anschlagelement des Gelenkfu-ßes zusammenwirken kann und somit eine Überstreckung der Prothese verhindert. Die bereits zuvor beschriebene Verlagerung der Gelenkachse leicht nach dorsal (also zur Rückseite hin) trägt dazu bei, hinsichtlich der Materialverlagerung, den anatomischen Gegebenheiten zu entsprechen und zudem eine möglichst gute Nachbildung der Kniegelenksbewegung zu erzielen.

Bei (teilweise) sphärischer Ausbildung der Gleitflächen kann der Streckanschlag ferner verhindern, dass in Streckstellung der Gelenkfuß senkrecht zur Gelenkachse zusätzlich rotierbar ist. Mit anderen Worten ist die (begrenzte) Rotation des Gelenkfußes um die Tibiaachse und somit relativ zum Gelenkkopf um eine Achse senkrecht zur Gelenkachse nur dann möglich, wenn die Gelenkeinrichtung eine Beugestellung (und somit die Funktionsstellung einer Kniebeugung) einnimmt, was den physiologischen Gegebenheiten entspricht.

Ferner kann die Gelenkeinrichtung dazu ausgebildet sein, ausgehend von einer Streckstellung eine Beugebewegung von bis zu 120° zu realisieren.

Die Prothese kann ferner ein femorales Knochenschaftersatzelement und/oder ein tibiales Knochenschaftersatzelement umfassen. Der Gelenkkopf kann hierbei mit dem distalen Ende des femoralen Knochenschaftersatzelements verbunden sein oder eine Einheit bilden. Ferner kann der Gelenkfuß mit dem proximalen Ende des tibialen Knochenschaftersatzelements verbunden sein oder eine Einheit bilden. Das femorale Knochenschaftersatzelement und/oder das tibiale Knochenschaftersatzelement können jeweils eine Durchgangsöffnung aufweisen. Die jeweilige Durchgangsöffnung des femoralen Knochenschaftersatzelements und/oder des tibialen Knochenschaftersatzelements kann mit dem wenigstens einen Führungskanal des Formkörpers und der Durchgangsöffnung des jeweiligen Gelenkanteils (Gelenkkopfs bzw. Gelenkfußes) fluchten, wenn die Gelenkeinrichtung die Funktionsstellung einer Kniebeugung einnimmt und dabei ausgehend von der Streckstellung um einen vorgegebenen Winkel, so wie oben beschrieben gebeugt ist.

### KURZE BESCHREIBUNG DER FIGUREN

Weitere Vorteile und Aspekte der Erfindung werden anhand von Zeichnungen erläutert. Es zeigen:
- **Figur 1**: eine Außenansicht einer erfindungsgemäßen Prothese zum Ersatz des menschlichen Kniegelenks in leichter Beugestellung;
- **Figur 2**: eine erste Variante der in der Figur 1 dargestellten Prothese in Explosionsdarstellung; und
- **Figur 3**: eine zweite Variante der in Figur 1 dargestellten Prothese in Explosionsdarstellung.

### DETAILLIERTE BESCHREIBUNG

Aspekte der vorliegenden Erfindung werden anhand exemplarischer Ausführungsformen weiter beschrieben.

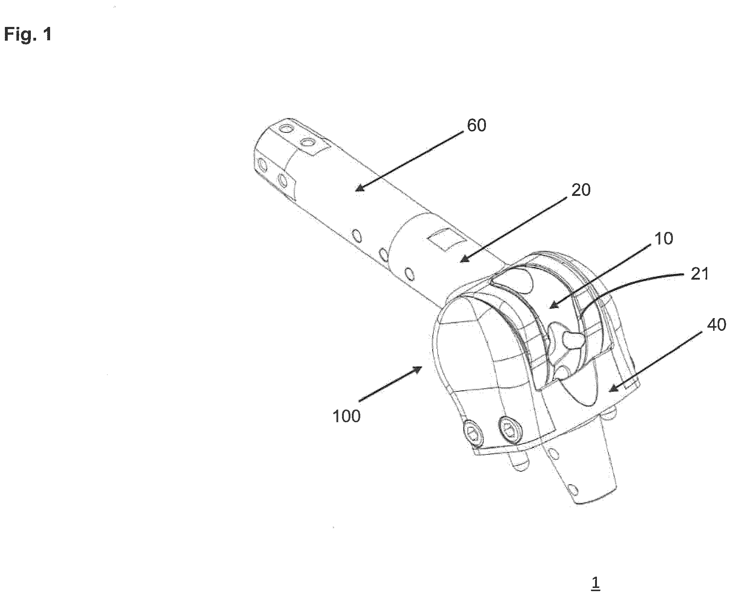

Figur 1 zeigt eine Außenansicht einer erfindungsgemäßen Prothese 1 in leichter Beugestellung.

Die Prothese 1 umfasst eine Gelenkeinrichtung 100 mit einem Gelenkkopf 20 und einem mit dem Gelenkkopf 20 gelenkig zusammenwirkenden Gelenkfuß 40. Ferner umfasst die Prothese 1 einen Formkörper 10. Der Formkörper 10 ist in einer mittig im Gelenkkopf 20 angeordneten Aussparung 21 entnehmbar eingesetzt. In der Figur 1 wird die Prothese 1 mit einem femoralen Knochenschaftersatzelement 60 gezeigt, das mit seinem distalen Ende mit dem Gelenkkopf 20 verbunden ist oder auch eine Einheit bilden kann. Ferner kann der Gelenkfuß 40 der Prothese 1 mit dem proximalen Ende eines tibialen Knochenschaftersatzelements (nicht gezeigt) verbunden sein oder auch eine Einheit bilden. Je nach Einsatz der Prothese 1 kann das femorale bzw. tibiale Knochenschaftersatzelement auch entfallen.

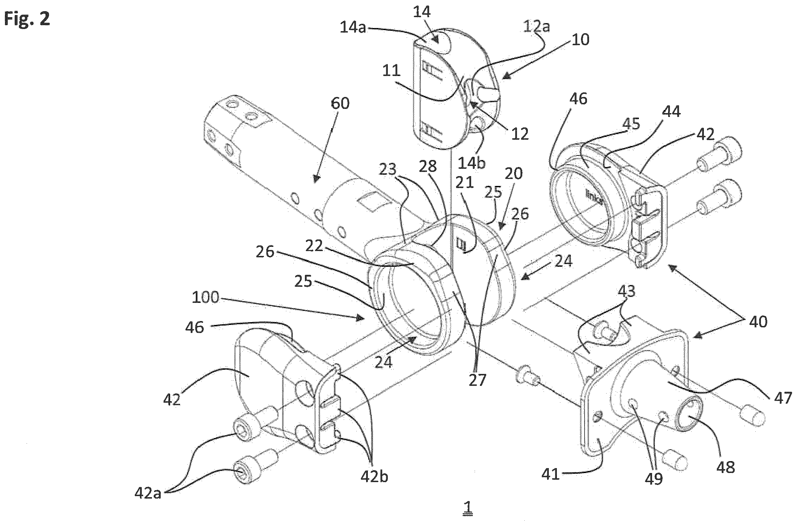

In Zusammenhang mit der Figur 2 wird eine erste Variante der Gelenkeinrichtung 100 weiter beschrieben.

Der Gelenkkopf 20 der in Figur 2 gezeigten Gelenkeinrichtung 100 ist walzenförmig (zylinderförmig) ausgebildet. Der Walzenkörper 22 definiert eine Rotationsachse, welche der Gelenkachse entspricht. Der Walzenkörper 22 weist mittig eine Aussparung 21 (auch Innenraum genannt) auf. Diese Aussparung 21 ist dergestalt, dass sie den Walzenkörper 22 in zwei Kopfabschnitte 23 teilt, wobei die Kopfabschnitte 23 durch die Aussparung 21 axial voneinander getrennt sind. Der Walzenkörper 22 geht an der von der Aussparung 21 abgewandten radialen Walzenaußenseite in einen massiven, zentralen Anteil des Gelenkkopfs 20 über, der mit dem femoralen Knochenschaftersatzelement 60 gekoppelt sein oder eine Einheit bilden kann oder direkt über eine Schaftverankerung mit dem Femur verbunden ist. Das femorale Knochenschaftersatzelement 60 kann eine Durchgangsöffnung aufweisen (in Figur 2 nicht sichtbar, da verdeckt), welche mit einer entsprechenden Durchgangsöffnung 28 des Gelenckopfs 20 bzw. des zentralen Anteils des Gelenkkopfs 20 fluchtet. Die Durchgangsöffnung 28 mündet in die Aussparung 21 des Gelenkkopfs 20 (siehe Figur 2).

An seinen gegenüberliegenden axialen Enden 24 weist der Gelenkkopf 20 jeweils einen ringförmigen Vorsprung 26 auf. Die beiden ringförmigen Vorsprünge 26 bilden jeweils einen Au-ßenring 26 an den gegenüberliegenden axialen Enden 24 des Walzenkörpers 22. Die Innenfläche 25 des jeweiligen Außenrings 26 bilden eine erste kreisrunde Gleitfläche 25 der Gelenkeinrichtung 100.

Ferner weist der Gelenkkopf 20 an seiner radialen Außenfläche wenigstens eine Anschlagfläche 27 auf. Die Anschlagfläche 27 ist an beiden Kopfabschnitten 23 ausgebildet. Konstruktionstechnisch kann die Anschlagfläche 27 dadurch realisiert werden, dass der walzenförmige oder hohlzylinderförmige Gelenkkopf 20 eine von einer kreis- oder walzenförmigen Körperform lokal abweichende Form aufweist. Wie aus den Figuren 1 und 2 ersichtlich ist, nimmt die radiale Ausdehnung des Gelenkkopfs 20 nach ventral hin (also zur Vorderseite hin) leicht zu. Diese zusätzliche radiale Ausdehnung oder Materialzunahme des Gelenkkopfs 20 bleibt in Umfangsrichtung des Gelenkkopfs 20 im Wesentlichen auf die Vorderseite des Gelenckopfs 20 beschränkt, so dass dort die in Figur 2 gezeigte Anschlagfläche 27 an beiden Kopfabschnitten 23 entsteht. Eine in Relation zur Femurschaftachse geringfügige Dorsalverlagerung der Gelenkachse des Gelenkkopfs 20 verhindert, dass sich die einseitige Materialzunahme (bzw. radiale Ausdehnung) des Gelenkkopfs 20 nach ventral anatomisch ungünstig auswirkt und optimiert die Gelenkeinrichtung 100 gemäß den Erfordernissen.

Der Gelenkfuß 40 der in Figur 2 gezeigten Gelenkeinrichtung 100 umfasst zwei seitlich angeordnete Gelenkelemente 42. Diese seitlichen Gelenkelementen 42 sind jeweils an einem zentral angeordneten Gelenkelement 41 axial beabstandet voneinander angeordnet. Der axiale Abstand der beiden seitlichen Gelenkelemente 42 ist hierbei derart gewählt, dass die seitlichen Gelenkelemente 42 relativ zu den beiden gegenüberliegenden axialen Enden 24 des Gelenkkopfs 20 axial weiter außen liegen. Somit wird der Gelenkkopf 20 zwischen den beiden seitlichen Gelenkelementen 42 um die Gelenkachse drehbar gelagert, wie weiter unten noch näher beschrieben wird.

Die seitlichen Gelenkelemente 42 können mit Hilfe von Schrauben 42a am zentralen Gelenkelement 41 des Gelenkfußes 40 lösbar befestigt sein. Andere Befestigungsmittel sind ebenso denkbar. An den beiden seitlichen Gelenkelementen 42 können ferner Justiernasen 42b vorgesehen sein, welche dazu ausgebildet sind, in entsprechende Justiernuten (in der Figur 2 nicht sichtbar) am zentralen Gelenkelement 41 formschlüssig einzugreifen. Dadurch wird die korrekte Montage, insbesondere die korrekte Ausrichtung der beiden lateralen Gelenkelemente 42 bezüglich des zentralen Gelenkelements 41 erleichtert. Ferner können die Justiernasen 42b als stabile Formschlusselemente ausgebildet sein, um Scherkräfte und Biegemomente von den Schrauben 42a abzuhalten, wodurch die mechanische Beständigkeit der Gelenkeinrichtung 100 weiter erhöht wird. Die Schrauben 42a sind demnach lediglich Axialkräften ausgesetzt.

Am zentralen Gelenkelement 41 ist ferner ein Anschlagelement 43 zwischen den beiden seitlichen Gelenkelementen 42 angeordnet. Es ist dazu ausgebildet, bei einer Streckbewegung mit der wenigstens einen Anschlagfläche 27 des Gelenkkopfs 20 in Anschlag zu kommen und somit eine Überstreckung des Gelenks zu verhindern. Mit anderen Worten wird mit Hilfe der wenigstens einen Anschlagfläche 27 am Gelenkkopf 20 und dem Anschlagelement 43 eine maximal mögliche Streckstellung der Gelenkeinrichtung 100 festgelegt.

Das zentrale Gelenkelement 41 kann an seiner von dem Anschlagelement 43 und den zwei seitlichen Gelenkelementen 42 abgewandten Seite mit einem (nicht gezeigten) tibialen Knochenschaftersatzelement verbunden sein. Der Gelenkfuß 40 weist einen Fixierungsfortsatz 47 auf, durch den eine Durchgangsöffnung 48 verläuft. Die Durchgangsöffnung 48 mündet wiederum in die Aussparung 21. Durch die Durchgangsöffnung 48 des Gelenkfußes 40 kann beispielsweise eine Schaftverankerung (in der Figur 1 nicht dargestellt), ein Werkzeug oder ein Distraktionsmarknagel zur Tibia hindurchgeführt werden. Insbesondere kann der Distraktionsmarknagel oder die tibiale Schaftverankerung mittels Befestigungsstiften am Fixierungsfortsatz 47 befestigt werden, indem die Befestigungsstifte durch in der Wandung des Fixierungsfortsatzes 47 vorgesehene Querbohrungen 49 geführt werden.

Die zwei seitlichen Gelenkelemente 42 bilden zusammen mit dem zentralen Gelenkelement 41 den gabelförmigen Gelenkfuß 40. Die beiden seitlichen Gelenkelemente 42 weisen jeweils eine axiale Innenfläche 44 auf, an denen jeweils ein ringförmiger Vorsprung 46 (im Folgenden auch Innenring 46 genannt) ausgebildet ist. Der jeweilige ringförmige Vorsprung 46 weist eine kreisförmige Außenfläche 45 auf, welche die zweite Gleitfläche 45 der Gelenkeinrichtung 100 bildet.

Im montierten Zustand greifen die jeweiligen Innenringe 46 der beiden seitlichen Gelenkelemente 42 in die jeweiligen Außenringe 26 des Gelenkkopfs 20 ein. Dadurch kommen die kreisförmig gekrümmten zweiten Gleitflächen 45 der seitlichen Gelenkelemente 42 mit den kreisförmig gekrümmten ersten Gleitflächen 25 des Gelenkkopfs 20 in Berührung. Somit kann der Gelenkfuß 40 relativ zum Gelenkkopf 20 um die Gelenkachse gedreht (rotiert) werden und somit eine Beuge- oder Streckbewegung realisieren. Zur Verbesserung der Gleitfähigkeit können die jeweiligen Gleitflächen 25, 45 jeweils mit einer biokompatiblen Beschichtung versehen sein oder aus einer verschleißresistenten und reibungsmindernden Materialkomponente, wie sie aus dem Stand der Technik bekannt sind, gefertigt sein.

Die lösbare Montage der beiden seitlichen Gelenkelemente 42 am zentralen Gelenkelement 41 (diese erfolgt mit Hilfe der oben beschriebenen Schrauben 42a und Justiernasen 42b) ermöglicht eine flexible Montage der Gelenkeinrichtung 100 (und somit der Prothese 1) während des operativen Eingriffs. So kann die Montage der Gelenkeinrichtung 100 sowohl von medial als auch von lateral erfolgen, abhängig vom operativen Zugang. In beiden Montagevarianten kann die Prothese 1 beim operativen Eingriff zunächst vormontiert werden, wobei bei einem Einsetzen der Prothese 1 von der medialen/lateralen Seite zunächst das von der medialen/lateralen Seite abgewandte laterale/mediale Prothesenelement 42 am zentralen Gelenkelement 41 vormontiert wird. Nach dem Einsetzen der Prothese 1 kann das der medialen/ lateralen Seite zugewandte Prothesenelement 42 am zentralen Prothesenelement 41 befestigt werden, wodurch die Gelenkeinrichtung 100 geschlossen wird.

Durch die hier beschriebene Ausgestaltung der Gelenkeinrichtung 100, bei der die gelenkige Lagerung zwischen dem Gelenkkopf 20 einerseits und dem gabelförmigen Gelenkfuß 40 andererseits axial so weit wie anatomisch möglich nach außen verlagert ist, wird die Stabilität der Gelenkeinrichtung 100 insgesamt verbessert. Dem zentralen Mittenbereich des Gelenckopfs 20, in welchem die Aussparung 21 mit Formkörper 10 zur Durchführung von Werkzeug, Distraktionsmarknagel oder einer femoralen oder tibialen Schaftverankerung vorgesehen ist, kommt keine gelenkstabilisierende Funktion zu.

Die Gelenkeinrichtung 100 ist insgesamt dazu ausgebildet, eine Streck- und/oder Beugebewegung entsprechend einem natürlichen Kniegelenk durchzuführen. Dies geschieht durch Drehung/Drehbewegung des Gelenkfußes 40 relativ zum Gelenkkopf 20 um die Gelenkachse. Bevorzugt kann die Gelenkeinrichtung 100, ausgehend von einer Streckstellung, einen maximalen Beugungswinkel von 120° realisieren. Die Streckstellung wird bei der Drehbewegung dann erreicht, wenn das zentrale Gelenkelement 41 des Gelenkfußes 40 an der wenigstens einen Anschlagfläche 27 des Gelenkkopfs 20 in Anschlag kommt.

Im Folgenden wird der Formkörper 10 weiter beschrieben. Der Formkörper 10 ist derart dimensioniert, dass er formschlüssig in die Aussparung 21 des Gelenkkopfs 20 eingesetzt werden kann. Der Formkörper 10 weist eine radial nach außen gekrümmte Oberfläche 11 auf, die einen Teil der Gleitfläche für die Kniescheibe realisiert, wenn der Formkörper 10 in der Aussparung 21 eingesetzt ist.

Im Formkörper 10 sind ferner zwei Durchgangsbohrungen 12, 14 realisiert, die sich im Inneren des Formkörpers 10 unter einem vorgegebenen Winkel kreuzen. Die erste Durchgangsbohrung 12 weist am Formkörper 10 eine erste Öffnung 12a und eine zweite Öffnung (in Figur 2 nicht sichtbar, da verdeckt) auf. Diese ist an der dem Prothesenkopf zugewandten Seite des Formkörpers 10 derart angeordnet, dass die Bohrung 12 mit der Durchgangsöffnung 28 des Gelenkkopfs 20 in Flucht kommt, wenn die Prothese 1 bzw. ihre Gelenkeinrichtung 100 eine einer Kniebeugung entsprechende vorgegebene Beugestellung (vorgegebenen Beugungswinkel) realisiert. Somit kann über einen kleinen operativen Zugang unterhalb der Kniescheibe und die erste Öffnung 12a beispielsweise ein Werkzeug, ein Distraktionsmarknagel oder eine Schaftverankerung durch die Durchgangsbohrung 12 und die sich anschließende Durchgangsöffnung 28 des Gelenkkopfs 20 und ggf. durch das femorale Knochenschaftersatzelement 60 in das Femur eingeführt oder entnommen werden, ohne die Gelenkeinrichtung 100 entkoppeln zu müssen.

Die zweite Durchgangsbohrung 14 weist am Formkörper 10 eine erste Öffnung 14a an der radial nach außen gekrümmten Oberfläche 11 sowie eine gegenüberliegende zweite Öffnung 14b auf. Die zweite Durchgangsbohrung 14 ist derart im Formkörper 10 angeordnet, dass die Bohrung 14 mit ihrer zweiten Öffnung 14b in Flucht mit der Durchgangsöffnung 48 des Gelenkfußes 40 und ggf. des tibialen Knochenschaftersatzelements kommt (in Figur 2 nicht dargestellt), wenn die Prothese 1 bzw. ihre Gelenkeinrichtung 100 eine einer Kniebeugung entsprechende vorgegebene Beugestellung (vorgegebenen Beugungswinkel) realisiert. Somit kann über einen kleinen operativen Zugang oberhalb der Kniescheibe durch die erste Öffnung 14a der zweiten Durchgangsbohrung 14 beispielsweise ein Werkzeug, ein Distraktionsmarknagel oder eine Schaftverankerung durch die Durchgangsbohrung 14 und die sich anschließende Durchgangsöffnung 48 des Gelenkfußes und ggf. durch das tibiale Knochenschaftersatzelement in die Tibia eingeführt oder entnommen werden, ohne die Gelenkeinrichtung 100 entkoppeln zu müssen. Insgesamt fungieren die erste Durchgangsbohrung 12 und die zweite Durchgangsbohrung 14 jeweils als Führungskanal zur Einführung oder Entnahme eines Werkzeugs, eines Distraktionsmarknagels oder einer Schaftverankerung.

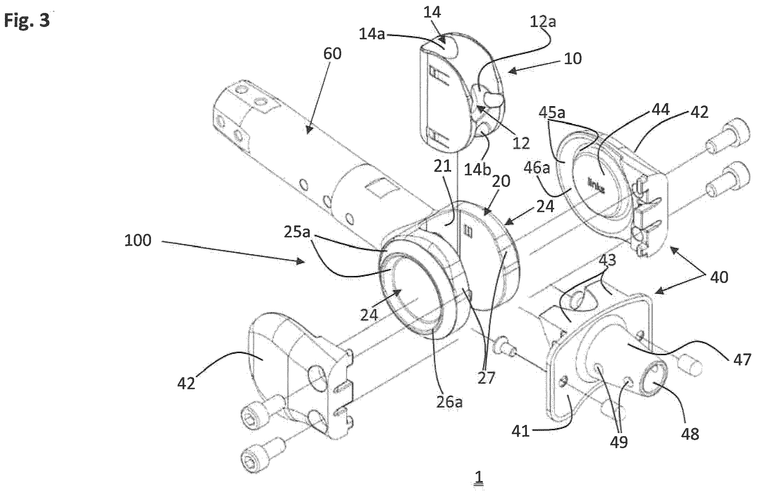

In Zusammenhang mit der Figur 3 wird eine zweite Variante der Gelenkeinrichtung 100 der Prothese 1 beschrieben.

Die Gelenkeinrichtung 100 gemäß Figur 3 unterscheidet sich von der Gelenkeinrichtung 100 gemäß Figur 2 in der Ausbildung der ersten und zweiten Gleitflächen 25a, 45a und somit in der gelenkigen Kopplung zwischen dem Gelenkkopf 20 und dem Gelenkfuß 40. Alle anderen Merkmale der Prothese 1 sind von ihrem strukturellen Aufbau und ihrer funktionellen Wirkungsweise gleich, so dass auf die Beschreibung der Figuren 1 und 2 verwiesen wird, um unnötige Wiederholungen zu vermeiden.

Im Unterschied zur Ausführungsform in der Figur 2, weist der Gelenkkopf 20 an seinen gegenüberliegenden axialen Enden 24 jeweils einen ringförmigen Vorsprung 26a mit einer konvex gewölbten, sphärischen Oberfläche 25a auf. Die konvex gewölbte, sphärische Oberfläche 25a fungiert als erste Gleitfläche 25a des Gelenkkopfs 20. Ferner weisen die zwei axial beabstandet angeordneten seitlichen Gelenkelemente 42 des gabelförmigen Gelenkfußes 40 an ihren axialen Innenflächen 44 jeweils eine zu den ringförmigen Vorsprüngen 26a korrespondierende ringförmige Nut 46a auf. Die jeweilige ringförmige Nut 46a weist eine konkav gewölbte, sphärische Oberfläche 45a auf, die als zweite Gleitfläche 45b fungiert.

Im montierten Zustand greifen die an den axialen Enden 24 des Gelenkkopfs 20 angeordneten ringförmigen Vorsprünge 26a in die an den axialen Innenflächen 44 der seitlichen Gelenkelemente 42 angeordneten und den ringförmigen Vorsprüngen 26a gegenüberliegenden ringförmigen Nuten 46a ein. Dadurch kommen die sphärisch konvex ausgebildeten ersten Gleitflächen 25a des Gelenkkopfs 20 mit den jeweiligen sphärisch konkav ausgebildeten zweiten Gleitflächen 45a des Gelenkfußes 40 in gleitende Berührung. Durch diese Ausgestaltung wird nicht nur eine Drehbewegung um die Gelenkachse möglich, sondern zusätzlich auch eine begrenzte Rotation (um einige Grad) um eine senkrecht zur Gelenkachse angeordnete und durch den Gelenkkfuß 40 verlaufenden Rotationsachse. Diese zusätzliche Rotation ist insbesondere dann möglich, wenn die Gelenkeinrichtung 100 sich in einer Beugestellung befindet. Befindet sich hingegen die Gelenkeinrichtung 100 in voller Streckstellung, bei der das Anschlagelement 43 des Gelenkfußes 40 mit der wenigstens einen Anschlagfläche 27 des Gelenkkopfs 20 im Anschlag ist, wird diese zusätzliche Rotation durch das Zusammenwirken von Anschlagelement 43 mit der wenigstens einen Anschlagfläche 27 unterbunden. Durch die hier beschriebene zusätzliche leichte Rotation senkrecht zur Gelenkachse in Beugestellung und die Aufhebung der Rotation in Streckstellung kann die Beweglichkeit eines Kniegelenks noch physiologischer nachgebildet werden, indem bei abgewinkelter Kniestellung eine begrenzte rotatorische Bewegung um die Tibiaachse möglich ist.

### Bezugszeichenliste

- 1: Prothese
- 10: Formkörper
- 11: Oberfläche Formkörper
- 12: erste Durchgangsbohrung/erster Führungskanal
- 12a: erste Öffnung
- 14: zweite Durchgangsbohrung/zweiter Führungskanal
- 14a: erste Öffnung
- 14b: zweite Öffnung
- 20: Gelenkkopf
- 21: Aussparung (Innenraum)
- 22: Walzenkörper
- 23: Kopfabschnitte
- 24: axiale(s) Ende(n)
- 25, 25a: erste Gleitfläche(n)
- 26: Außenring
- 26a: ringförmiger Vorsprung
- 27: Anschlagfläche(n)
- 28: Durchgangsöffnung Gelenkkopf
- 40: Gelenkfuß
- 41: zentrales Gelenkelement
- 42: seitliche(s) Gelenkelement(e)
- 42a: Schrauben
- 42b: Justiernasen
- 43: Anschlagelement
- 44: axiale Innenfläche(n) des(der) seitlichen Gelenkelements(e)
- 45, 45a: zweite Gleitfläche(n)
- 46: Innenring
- 46a: ringförmige Nut
- 47: Fixierungsfortsatz
- 48: Durchgangsöffnung Gelenkfuß
- 49: Querbohrung
- 60: femorales Knochenschaftersatzelement
- 100: Gelenkeinrichtung

## Patentansprüche

1. Implantierbare Prothese (1) zum Ersatz eines menschlichen Kniegelenks, umfassend eine Gelenkeinrichtung (100) mit einem Gelenkkopf (20) und einen mit dem Gelenkkopf (20) zusammenwirkenden Gelenkfuß (40), die zur wahlweisen Realisierung wenigstens einer Gelenkbewegung in Form einer Beuge- und/oder Streckbewegung vorgesehen sind, wobei der Gelenkkopf (20) zwei erste Gleitflächen (25, 25a) aufweist, die an gegenüberliegenden axialen Enden (24) des Gelenkkopfs (20) angeordnet sind und durch einen mittig im Gelenkkopf (20) angeordneten Innenraum (21) räumlich voneinander getrennt sind,
wobei der Gelenkfuß (40) zwei zweite Gleitflächen (45, 45a) aufweist, die an dem Gelenkfuß (40) axial gegenüberliegend ausgebildet sind und mit jeweiligen ersten Gleitflächen (25, 25a) des Gelenkkopfs (20) in Berührung kommen, wobei die zwei ersten Gleitflächen (25, 25a) und die zwei zweiten Gleitflächen (45, 45a) zur Realisierung wenigstens einer Beuge- und/oder Streckbewegung ausgebildet sind.

2. Implantierbare Prothese (1) nach Anspruch 1, wobei der Gelenkfuß (40) zur beweglichen Aufnahme des Gelenkkopfs (20) ausgebildet ist.

3. Implantierbare Prothese (1) nach Anspruch 1 oder 2, wobei der Gelenkkopf (20) im Wesentlichen walzenförmig ausgebildet ist.

4. Implantierbare Prothese (1) nach einem der Ansprüche 1 bis 3, wobei der Innenraum (21) den Gelenkkopf (20) im Wesentlichen in zwei axiale Kopfabschnitte (23) unterteilt.

5. Implantierbare Prothese (1) nach einem der Ansprüche 1 bis 4, ferner umfassend einen Formkörper (10), der zur Aufnahme im Innenraum (21) des Gelenkkopfs (20) vorgesehen ist.

6. Implantierbare Prothese (1) nach Anspruch 5, wobei der Formkörper (10) wenigstens einen Führungskanal (12, 14) zur Durchführung eines Werkzeuges, eines Distraktionsmarknagels oder einer femoralen oder tibialen Schaftverankerung aufweist.

7. Implantierbare Prothese (1) nach Anspruch 6, wobei der wenigstens eine Führungskanal (12, 14) im Formkörper (10) derart ausgerichtet angeordnet ist, dass ein Distraktionsmarknagel oder eine femorale oder tibiale Schaftverankerung bei einem vorgegebenen Beugungswinkel durch die Gelenkeinrichtung (100) in ein angrenzendes femorales Knochenschaftersatzelement (60) und/oder tibiales Knochenschaftersatzelement durchgeführt werden kann.

8. Implantierbare Prothese (1) nach einem der Ansprüche 1 bis 7, wobei die zwei ersten Gleitflächen (25) und die zwei zweiten Gleitflächen (45) jeweils kreisförmig radial gekrümmte Gleitflächen sind.

9. Implantierbare Prothese (1) nach einem der Ansprüche 1 bis 7, wobei die zwei ersten Gleitflächen (25a) und die zwei zweiten Gleitflächen (45a) jeweils kreisförmig sphärisch gekrümmte Gleitflächen sind.

10. Implantierbare Prothese (1) nach Anspruch 9, wobei die zwei ersten Gleitflächen (25a) und die zwei zweiten Gleitflächen (45a) dazu ausgebildet sind, zusätzlich zu der Beuge- und/oder Streckbewegung um die Gelenkachse eine dazu senkrechte, begrenzte Rotationsbewegung zu ermöglichen, wenn die Gelenkeinrichtung (100) sich in einer Beugestellung befindet.

11. Implantierbare Prothese (1) nach einem der Ansprüche 1 bis 10, wobei die zwei ersten Gleitflächen (25, 25a) und/oder die zwei zweiten Gleitflächen (45, 45a) eine biokompatible Beschichtung aufweisen oder aus einer zusätzlichen Materialkomponente zur Reibungsverminderung bestehen.

12. Implantierbare Prothese (1) nach einem der Ansprüche 1 bis 11, wobei die Gelenkeinrichtung (100) ferner eine Gleitfläche für eine Kniescheibe aufweist.

13. Implantierbare Prothese (1) nach einem der Ansprüche 1 bis 12, wobei die Gelenkeinrichtung (100) ferner einen Streckanschlag umfasst, welcher zur Begrenzung der Streckbewegung vorgesehen ist, wobei der Streckanschlag durch wenigstens eine am Gelenkkopf (20) ausgebildete Anschlagfläche (27) realisiert ist.

14. Implantierbare Prothese (1) nach einem der Ansprüche 1 bis 13, wobei die Gelenkeinrichtung (100) dazu ausgebildet ist, ausgehend von einer Streckstellung eine Beugebewegung von bis zu 120° zu realisieren.

15. Implantierbare Prothese (1) nach einem der Ansprüche 1 bis 14, wobei die Prothese (1) ferner ein femorales Knochenschaftersatzelement (60) und/oder ein tibiales Knochenschaftersatzelement umfasst, wobei der Gelenkkopf (20) mit dem distalen Ende des femoralen Knochenschaftersatzelements (60) verbunden ist oder eine Einheit bildet, wobei der Gelenkfuß (40) mit dem proximalen Ende des tibialen Knochenschaftersatzelements verbunden ist oder eine Einheit bildet.
